# EUROPEAN PATENT APPLICATION

(11) **EP 0 736 599 A2**
(43) Date of publication of application: **09.10.1996**
(21) Application number: 96105353.5
(22) Date of filing: 03.04.1996
(51) Int. Cl.: C12N 15/12, C12N 1/21, C07K 14/47, C07K 16/18

(54) **Rat obese gene, its gene product and its production**

(30) Priority: 03.04.1995 JP 77966/95
(71) Applicant: Takeda Chemical Industries, Ltd., Osaka-shi, Osaka 541 (JP)
(72) Inventor: Nakao, Kazuwa, Kyoto 610-11 (JP); Ogawa, Yoshihiro, Osaka 569 (JP); Fujisawa, Yukio, Kobe, Hyogo 658 (JP)
(74) Representative: KUHNEN, WACKER & PARTNER

(57) **Abstract**

A polypeptide which has an amino acid sequence comprising the amino acid sequence shown in SEQ ID NO: 1 or 2; a recombinant DNA encoding the polypeptide; a vector comprising the recombinant DNA; a host cell transformed with the recombinant DNA; a method for producing the polypeptide, which comprises the steps of culturing the host cell in a culture medium, accumulating the polypeptide in the culture and recovering the same; and an antibody which reacts to a gene product coding for rat obese cDNA.

## Description

### FIELD OF THE INVENTION

The present invention relates to a novel rat obese gene product, a recombinant DNA encoding the product, a transformant carrying the DNA, and production and use of the product. Specifically, the present invention relates to a rat-derived obese gene prepared by recombinant DNA techniques, and its substantially pure gene product expressed in prokaryotic or eukaryotic cells. The gene product of the invention can be used for the production of antibodies to the gene product or for the screening of receptors.

### BACKGROUND OF THE INVENTION

The mechanisms that maintain equilibrium between food intake and energy expenditure determine the extent of obesity and leanness. Because excessive obesity aggravates type II (non-insulin-dependent) diabetes mellitus, hypertension, hyperlipemia or certain cancers, obesity has become an important problem in health maintenance. It is known that mice having obese phenotypes have five single gene mutations (J. M. Fridman and R. L. Leibel, Cell, vol. 69, p. 217 (1992)). Of them, an obese gene (hereinafter sometimes referred to as ob gene) is one of the molecules that control energy balance. Its mutation disorders the mechanisms controlling the energy balance, thereby inducing human type II diabetes mellitus or serious obesity - like symptoms (J. M. Friedman et al., Genomics, vol. 11, p. 1054 (1991)). Parabiosis experiments between ob gene - mutated mice and wild type mice showed that ob gene mice lack a blood-derived factor that controls nutriment intake and metabolism (D. L. Coleman, Diabetologia, vol. 14, p. 141 (1978)). However, detailed properties of this factor remain unclear.

Recently, J. M. Friedman et al. first cloned mouse ob genes by the positional cloning technique (Y. Zhang et al., Nature, vol. 372, p. 425 (1994)). The results demonstrate that the mouse ob gene has an open reading frame of 167 amino acid residues and its mRNA is expressed specifically in fat tissues. It is believed that the product encoded by the mouse ob gene has a signal sequence composed of 21 amino acid residues in the N-terminus and has properties of secretory proteins (Y. Zhang et al., Nature, vol. 372, p. 425 (1994)). At the same time, a human gene corresponding to the mouse ob gene was cloned. It is reported that the human and mouse presumptive amino acid sequences are 84% identical at the amino acid level. It is presumed that the protein encoded by ob gene plays important roles in signal transmission mechanisms that control body fat amounts. In addition, when a human ob gene is transcribed to RNA using SP6 polymerase and the resulting RNA is translated in vitro in the presence of microsomal membrane fractions, the product in which 16K has been processed and the primary translation product of 18K are produced in nearly equal amounts. When this translation product is treated by proteinase K, the 18K primary translation product undergoes complete proteolysis, whereas the 16K-processed product is not affected. This fact shows that the translation product has been translocated to the microsomal lumens. When Triton X-100 is added to the system to make the membrane permeable, the 16K-processed product is made sensitive to proteases. These results show that the ob gene product is a secretory protein. It is presumed that two cysteine residues remain in the mature protein after the signal sequence is decomposed. Therefore the ob gene product may contain disulfide bonds like other secretory polypeptides. This ob gene product has no characteristic structural motif or membrane spanning domains other than the N-terminal signal sequence. In addition, the consensus sequences of dibasic amino acid sequences showing N-bound glycosylation or proteolysis sites have not been found.

As described above, the mouse- and human-derived ob genes have been cloned. However, no rat-derived ob genes have been obtained. To elucidate the role of the gene in obesity control, it is important to establish a detection system of the gene product. To prepare antibodies used for the detection, it is essential to establish an expression system providing a large amount of the gene product. In addition, in terms of handling in animal experiments, it is desired to establish an animal experimental model using rats. For this purpose, it is necessary to obtain rat-derived ob genes. Thus, there is a need for a process for cloning the rat-derived ob gene, constructing a transformant which expresses the gene, and preparing the ob gene product by using the transformant.

### OBJECTS OF THE INVENTION

The main object of the present invention is to provide a rat obese gene, its gene product and a process for producing the gene product.

This object as well as other objects and advantages of the present invention will become apparent to those skilled in the art from the following description with reference to the accompanying drawings.

### BRIEF DESCRIPTION OF THE DRAWINGS

Fig. 1 shows the results of Western blotting in Example 3. The term "kDal" represents kilodalton.

Fig. 2 shows the results of Western blotting in Example 4. The term "KDal" represents kilodalton.

### SUMMARY OF THE INVENTION

As a result of intensive studies, the present inventors have succeeded in cloning a gene having a high homology to a mouse ob gene cDNA from a rat fat cell cDNA library and preparing the gene product from cells transformed with the gene.

The present invention provides a polypeptide which has an amino acid sequence comprising the amino acid sequence shown in SEQ ID NO: 1 or 2 described below. The polypeptide is preferably a gene product coding for rat obese cDNA.

The present invention also provides a recombinant DNA encoding the above polypeptide. The recombinant DNA preferably comprises the DNA sequence shown in SEQ ID NO: 3 or 4 described below.

The present invention also provides a vector comprising the above recombinant DNA.

The present invention also provides a host cell transformed with the above recombinant DNA. The host cell is preferably an Escherichia coli cell.

The present invention also provides a method for producing the above polypeptide, which comprises the steps of culturing the above host cell in a culture medium, accumulating the polypeptide in the culture and recovering the same.

The present invention also provides an antibody which reacts to a gene product coding for rat obese cDNA.

### DETAILED DESCRIPTION OF THE INVENTION

In the present invention, the polypeptide of SEQ ID NO: 1 or 2 is encoded, for example, by the DNA comprising the base sequence of SEQ ID NO: 3 or 4 obtained in Examples below. Examples of the polypeptide include translation products of a rat-derived obese gene (ob).

The base sequence analysis of the DNA containing the base sequence of SEQ ID NO: 4 obtained in Examples below from the rat fat cell cDNA library showed a 95.6% homology to the mouse ob gene cDNA reported so far at the DNA level. From this result, it is believed that the DNA containing the base sequence of SEQ ID NO: 4 is a rat ob gene. The polypeptide composed of the amino acid sequence translated from the base sequence of the rat ob gene is a rat ob gene product.

In the present invention, the polypeptide composed of the amino acid sequence of SEQ ID NO: 1 that is considered to be an essential region because of its activity as the ob gene product may have about 1 to 50 amino acid residues before or after the polypeptide. That is, the polypeptides of the invention include polypeptides having about 140 to 200 amino acid residues. Another polypeptide composed of about 10 to 300 amino acid residues may be attached before the N-terminus of the polypeptide to form a fused protein. Such a fused protein can be used in the invention.

The polypeptide composed of the amino acid sequence of SEQ ID NO: 1 or 2 may have substitutions or deletions of amino acid residues in the sequence so long as the polypeptide maintains its activity as the ob gene product (e.g., affinity to anti - ob gene product antibody).

The polypeptide of SEQ ID NO: 2 obtained in the invention showed a 96.4% homology at the amino acid sequence level to the polypeptide obtained as the translation product of the mouse ob gene cDNA reported so far. However, the amino acid residues different between the two polypeptides are not particularly localized, but observed universally in the polypeptide sequence. Therefore it is considered that the two polypeptides have different properties from each other.

The recombinant DNA of the invention can be prepared by preparing RNA and synthesizing its cDNA according to the following procedure.

The RNA encoding the mouse-derived ob gene obtained in the invention can be obtained from rat-derived fat tissues. The RNA can be prepared from these materials by, for example, the guanidine thiocyanate method (J. M. Chirgwin et al., Biochemistry, vol. 18, p. 5294 (1979)), etc.

Oligo dT primers or random oligonucleotides are added to the RNA thus obtained, and then reverse transcriptase is added to synthesize the cDNA. Sense and antisense primers to amplify the rat-derived ob gene from this cDNA sample based on the mouse ob gene sequence reported so far (Zhang, Y. et al., Nature, vol. 372, p. 425 (1994)) are added to amplify the desired rat-derived ob gene cDNA according to a known PCR method (see e.g. the instruction for the kit of Cetus/Perkin-Elmer). The cDNA thus amplified is separated by per se known methods (e.g., agarose gel electrophoresis), recovered from the gel, and introduced into a plasmid vector by per se known methods to construct a recombinant expression plasmid. In this manner, the cDNA can be cloned. The base sequence of the desired cDNA can be determined by, for example, the dideoxynucleotide synthesis chain termination method (T. Messing et al., Nucl. Acids Res., vol. 9, p. 309 (1981)).

The plasmid containing the cloned cDNA can be used as it is or, if necessary, after it is cleaved with an appropriate restriction enzyme to insert it into another vector.

The plasmid vector is not specifically limited so long as it can be replicated in the host containing the plasmid vector. When the host is Escherichia coli, examples of the plasmid vectors include Escherichia coli - derived plasmids such as pBR 322 (F. Bolivar et al., Gene, vol. 2, p. 95 (1979)), pBR325, pUC12, pUC13, etc. When the host is yeast, examples of the plasmid vectors include yeast-derived plasmids such as pSH19 (S. Harashima et al., Mol. Cell. Biol., vol. 4, p. 771 (1984)), pSH19-1 (EP-A-0235430), etc. When the host is an animal cell, examples of the plasmid vectors include pSV2-X obtained by inserting ori of SV40 into pBR322 (R. C. Mulligan and P. Berg, Proc. Natl. Acad. Sci. USA, vol. 78, p. 2072 (1981)), pcD-X (H. Okayama and P. Berg, Mol. Cell. Biol., vol. 3, p. 280 (1983)), etc. When the host is an insect cell, examples of the plasmid vectors include Baculoviurus transfer vector pVL1392, pVL1393 (manual (MAXBAC™ Baculovirus expression system, Manual version 1.4) of the manufacturer Invitrogen Corporation, CA, USA), etc.

The cloned cDNA may have a translation initiation codon (ATG) at the 5'-terminus or a translation termination codon (TAG, TGA or TAA) at the 3'-terminus. In addition, a promoter sequence may be attached to the upstream region to express the cDNA.

The above promotor is not specifically limited so long as it can appropriately function in the host to be used for the gene expression. When the host is Escherichia coli, the promoters include T7 promoter, trp promoter, tac promoter, lac promoter, γPL promoter, etc. In particular, T7 promoter is preferred because of its high expression efficiency for the desired gene. When the host is yeast, the promoters include GAPDH promoter, PGK promoter, PHO5 promoter, ADH promoter, etc. In particular, GAPDH promoter is preferred because of its high expression efficiency for the desired gene. When the host is an animal cell, the promoters include SV40-derived promoter, retrovirus promoter, human cytomegalovirus promoter, etc. When the host is an insect cell, the promoters include polyhedrin promoters of nuclear-polyhedrosis virus, etc.

The promoter peculiar to the corresponding gene can be used as it is. Promoters chemically synthesized with DNA synthesizers, etc., can also be used.

The signal sequence and pre-pro sequence to be used are preferably those peculiar to the ob gene. However, any signal sequences and pre-pro sequences can be used so long as they can function in the host. The signal sequence and pre-pro sequence may be those chemically synthesized with DNA synthesizers, etc.

The recombinant expression plasmid containing the desired DNA thus constructed can be introduced into a host by a known method to prepare a transformant.

Examples of the hosts include bacteria belonging to the genus Escherichia, yeast, animal cells, insect cells, etc. The bacteria belonging to the genus Escherichia include Escherichia coli K12 DH1 (B. Low, Proc. Nat. Acad. Sci. USA, vol. 60, p. 160 (1968)), C600 (R. K. Appleyard, Genetics, vol. 39, p. 440 (1954)), MM294 (K. Backman et al., Proc. Natl. Acad. Sci. USA, vol. 73, p. 4174 (1976)), N4830 (M. E. Gottesman et al., J. Mol. Biol., vol. 140, p. 57 (1980)), etc. The yeast includes, for example, Saccharomyces cerevisiae AH22R⁻ (A. Miyanohara et al., Proc. Nat. Acad. Sci. USA, vol. 80, p. 1 (1983)), NA87-11A, DKD-5D, NA-74-3A, NA74-3Aρ⁻ (Y. Kaisho et al., Yeast, vol. 5, p. 91 (1989)); Schizosaccharomyces pombe ATCC38399 (h-leul-32), TH168 (h90 ade6-M210 ural leul) (M. Kishida and C. Shimada, Current Genetics, vol. 10, p. 443 (1986)), etc. The animal cells include, for example, adherent cells such as simian COS-7 cells, simian Vero cells, chinese hamster ovarian (CHO) cells, mouse L cells and human FL cells; suspended cells such as mouse myeloma cells (Sp 2/0 cell, etc.), mouse YAC-1 cells, mouse Meth A cells, mouse P388 cells, mouse EL-4 cells, etc. The insect cells include Sf9 cells, etc.

The transformant can be constructed by a known method.

Bacteria belonging to the genus Escherichia can be transformed, for example, by the method of T. Maniatis et al. (Molecular Cloning, Cold Spring Harbor Laboratory, p. 249 (1982)). Yeast can be transformed, for example, by the method of A. Hinnen et al. (proc. Natl. Acad. Sci. USA, vol. 75, p. 1929 (1978)). Animal cells can be transformed, for example, by the method of M. Wigler et al. (Cell, vol. 14, p. 725 (1978)). Insect cells can be transformed according to the manual (MAXBAC™ Baculovirus expression system, Manual version 1.4) of the manufacturer Invitrogen Corporation.

The transformant thus obtained can be cultivated by a per se known method.

When the host is a bacterium belonging to the genus Escherichia, the resulting transformant is preferably cultivated, for example, in M9 medium containing glucose, casamino acids (J. H. Miller, Experiments in Molecular Genetics, p. 431, Cold Spring Harbor Laboratory (1972)). If necessary, chemicals such as isopropylthiogalactoside (IPTG) and indolyl-3-acrylic acid can be added to efficiently operate the operator. The cultivation is normally carried out normally at about 15 to 43°C for about 3 to 24 hours, if necessary, with aeration or shaking.

When the host is yeast, the resulting transformant is cultivated, for example, in Burkholder minimal medium (K. L. Bostain et al., Proc. Natl. Acad. Sci. USA, vol. 77, p. 4504 (1980)), etc., as the host. The pH of the medium is preferably adjusted to about 5 to 8. The cultivation is normally carried out at about 20 to 35°C for about 24 to 72 hours, if necessary, with aeration or shaking.

When the host is an animal cell, the resulting transformant is cultivated, for example, in MEM medium containing about 5 to 20% fetal calf serum (H. Eagle, Science, vol. 130, p. 432 (1959)), DMEM medium (R. Dulbecco and G. Freeman, Virology, vol. 8, p. 396 (1959)), RPMI-1640 medium (G. E. More et al., J. Am. Med. Assoc., vol. 199, p. 519 (1967)), 199 medium (J. F. Morgan et al., Proc. Soc. Exp. Biol. Med., vol. 73, p. 1 (1950)), ASF104 medium (Ajinomoto), etc. The cultivation is normally carried out at 30 to 40°C for about 15 to 60 hours, if necessary, with aeration or shaking.

When the host is an insect cell, the resulting transformant is cultivated, for example, in TNM-FH medium (W. F. Hink et al., Nature, vol. 226, p. 466 (1990)), etc. The cultivation is normally carried out at about 15 to 30°C for about 24 to 72 hours, if necessary, with aeration or shaking.

In the present invention, the expression product can be isolated from the above culture by an appropriate combination of per se known separation and purification methods. The known separation and purification methods include methods utilizing solubility differences such as salting out, solvent precipitation, etc., methods mainly utilizing molecular weight differences such as dialysis, ultrafiltration, gel filtration and SDS-polyacrylamide gel electrophoresis (SDS-PAGE), etc., methods utilizing charge differences such as ion exchange chromatography, etc., methods utilizing specific affinity such as affinity chromatography, etc., methods utilizing hydrophobicity differences such as reverse phase high performance liquid chromatography, etc., methods utilizing isoelectric point differences such as isoelectric focusing, etc.

The rat ob gene obtained by the invention is expressed in prokaryotic or eukaryotic cells. The resulting ob gene product can be used to elucidate obesity mechanisms in rats and diagnose obesity. The gene obtained by the invention can be used as a DNA probe to determine the content of the ob gene mRNA expressed in the living body by the known Northern blotting method. The ob gene product obtained by the invention can be expressed in Escherichia coli, animal culture cells, insect culture cells, etc., to produce a large amount of the gene product in high purity.

In addition, the anti - ob gene product antibody obtained using the rat ob gene product obtained by the invention can be used to diagnose obesity by determining the product in the living body by the fluorescent antibody technique and Western blotting.

The abbreviations of bases, amino acids, etc., used in the specification or drawings are those established by the Commmision on Biochemical Nomenclature (CBN) or based on abbreviations commonly used in the art. Examples of the abbreviations are described below. When amino acids have optical isomers, they are L-isomers unless otherwise indicated.
- DNA :: Deoxyribonucleic acid
- A :: Adenine
- T :: Thymine
- G :: Guanine
- C :: Cytosine
- SDS :: Sodium dodecyl sulfate
- Gly :: Glycine (G)
- Ala :: Alanine (A)
- Val :: Valine (V)
- Leu :: Leucine (L)
- Ile :: Isoleucine (I)
- Ser :: Serine (S)
- Thr :: Threonine (T)
- Cys :: Cysteine (C)
- 1/2 Cys:: Half cystine
- Met :: Methionine (M)
- Glu :: Glutamic acid (E)
- Asp :: Aspartic acid (D)
- Lys :: Lysine (K)
- Arg :: Arginine (R)
- His :: Histidine (H)
- Phe :: Phenylalanine (F)
- Tyr :: Tyrosine (Y)
- Trp :: Tryptophan (W)
- Pro :: Proline (P)
- Asn :: Asparagine (N)
- Gln :: Glutamine (Q)
- Ap^{r} :: Ampicillin - resistant gene
- Tc^{r} :: Tetracycline - resistant gene

The following reference examples and examples further illustrate the present invention in detail, but are not to be construed to limit the scope of the invention.

Escherichia coli JM109/pBSK-rob carrying the plasmid pBSK-rob constructed in Example 1 below has been deposited at the Institute for Fermentation, Osaka (IFO) under the Accession Number IFO 15809 since March 17, 1995, and at the National Institute of Bioscience and Human-Technology, Agency of Industrial Science and Technology (1-3, Higashi 1 chome, Tsukuba-shi, Ibaraki-ken 305 Japan) under the Accession Number FERM BP-5075 on April 12, 1995 under the Budapest Treaty.

In the reference examples and examples, the DNA base sequences were determined by the known dideoxynucleotide synthesis chain termination method (J. Messing et al., Nucleic Acids Res., vol. 9, p. 309 (1981)).

### Reference Example 1

### Cloning of the obese gene (ob) cDNA from the cDNA library derived from mouse fat tissues

To amplify the ob gene cDNA by the PCR method, the following two primers were synthesized by referring to the reported base sequence of the ob gene cDNA derived from mouse fat tissues (Y. Zhang et al., Nature, vol. 372, p. 425 (1994)).
Sense Primer No. 1:
   5'-AATGTGCTGGAGACCCCTGT-3'
   (SEQ ID NO: 5)
Antisense Primer No. 2:
   5'-CAGCATTCAGGGCTAACATC-3'
   (SEQ ID NO: 6)

mRNA was extracted from the mouse epididymis fat tissues (Chomczynski, P. and Sacchi, N., Anal. Biochem. vol. 162, p. 156 (1987)), and single-stranded cDNA fragment was synthesized using Oligo (dT) primer. cDNA was synthesized using the above two primers (Nos. 1 and 2) and reverse transcriptase (Superscript Moloney murine leukemia virus reverse transcriptase, Bethesda Research Laboratories Inc., Gaithersburg, MD, USA), and used as the template for the PCR method. The above two primers (Nos. 1 and 2; each 50 pmol) were added to this cDNA, and fifty PCR cycles (at 94°C for 1 minute, at 55°C for 2 minutes and at 72°C for 1 minute) were carried out using Ampli Taq DNA polymerase (Takara Shuzo Co., Ltd.). The PCR product was separated by 1.2% agarose gel electrophoresis. The amplified DNA fragments were observed at the site corresponding to the size (505 bp) predicted from the mouse fat tissue - derived ob gene cDNA base sequence. These DNA fragments were recovered from the gel, and subcloned into the plasmid vector pGEM^{R}-T vector (Promega Inc., WI, USA). The base sequence of the obtained cDNA was determined by the dideoxynucleotide synthesis chain termination method, and confirmed that it was identical with the sequence already reported. The plasmid containing this cDNA was designated as pGEM-mob.

### Reference Example 2

### Preparation of the [α-³²P]dCTP labelled probe of mouse ob gene cDNA

The PCR product of the mouse ob gene cDNA of Reference Example 1 was used as a template to prepare a probe labelled with [α-³²P]-dCTP (3,000Ci/mmol, Amersham International Buckinghamshire, UK) by the random primer method (J. Sambrook et al., Molecular Cloning: A Laboratory Manual, Cold Spring Harbor Laboratory, Chapter 10, p. 13 (1989)). The specific radioactivity was about 1 x 10⁹ cpm/µg.

### Example 1

### Cloning of rat ob gene cDNA

Escherichia coli (Y1090r⁻) was infected with the rat adipocyte cDNA library λgt11 (CLONTECH Laboratories, Inc.), inoculated in ten 90 mm diameter petri dishes, and cultivated at 37°C for 7 hours. Then, the phages were transferred to a Nylon filter (Hybond-N⁺, Amersham International Buckinghamshire, UK) to prepare a replica. Hybridization was carried out at 68°C using the probe prepared in Reference Example 2 (J. Sambrook et al., Molecular Cloning: A Laboratory Manual, Chapter 2, p. 108, Cold Spring Harbor Laboratory, New York (1989)) to obtain 4 positive clones. Base sequence analysis of each clone showed that there was no clone encoding the entire open reading frame (ORF) of the desired rat ob gene cDNA. The incomplete part of cDNA was obtained by the PCR method according to 5'-AmpliFINDER™ RACE kit (CLONTECH Laboratories, Inc.). That is, first, the following two-antisense primers were synthesized based on the base sequence of the above cDNA.
Antisense Primer No. 3:
   5'-ATCCTGGTGACAATGGTC-3'
   (SEQ ID NO: 7)
Antisense Primer No. 4:
   5'-CTGTTGATAGACTGCCAG-3'
   (SEQ ID NO: 8)

Then, mRNA was extracted from epidymis fat tissues of SD (Sprague-Dawley) rats (12 weeks old, female) (P. Chomczynski and N. Sacchi, Anal. Biochem., vol. 162, p. 156 (1987)), and a single-stranded cDNA fragment was synthesized using antisense primer No. 3. Using Ampli FINDER anchor primer and antisense primer No. 4, PCR was carried out on a template prepared by linking Ampli FINDER anchor to the above single-stranded cDNA fragment with T4 RNA ligase. The PCR reaction conditions were the same as in Reference Example 1. As a result, cDNA fragments containing the region encoding 184 bp rat ob gene cDNA were obtained. Base sequence analysis of the fragment showed a 95% homology to the mouse ob gene cDNA. Then, to obtain rat ob gene cDNA encoding the entire ORF of the rat ob gene, the following 4 primers were synthesized based on the base sequence of the cDNA fragment thus obtained.
Sense Primer No. 5:
   5'-TGCTCCAGCAGCTGCAAGGT-3'
   (SEQ ID NO: 9)
Sense Primer No. 6:
   5'-CAAGAGGAATTCCCCAGCGAGGAAA-3'
   (SEQ ID NO: 10)
Antisense Primer No. 7:
   5'-TGAGGATACCTGGGAGCCAA-3'
   (SEQ ID NO: 11)
Antisense Primer No. 8
   5'-GGGAATTCCCTCAACATGATCCTCG-3'
   (SEQ ID NO: 12)

Then, mRNA was extracted from epidymis fat tissues of SD (Sprague-Dawley) rats (12 weeks old, female) (P. Chomczynski and N. Sacchi, Anal. Biochem., vol. 162, p. 156 (1987)), and cDNA was synthesized from 10 µg of the total RNA using Oligo (dT) primer and a reverse transcriptase (Superscript Moloney murine leukemia virus reverse transcriptase, Bethesda Research Laboratories Inc., Gaithersburg, MD, USA), and used as a template for PCR. Two primers (the above Nos. 5 and 7; each 50 pmol) were added in the 1st PCR. Fifty PCR cycles (94°C for 1 minutes, 55°C for 2 minutes and 72°C for 1 minute) were carried out using Vent^{R} DNA polymerase (New England Biolabs, Inc., MA, USA). In addition, other 2 primers (the above Nos. 6 and 8; each 50 pmol) were added to the reaction mixture, and the reaction was carried out in the same manner. The PCR product was separated by 1.2% agarose gel electrophoresis to obtain the amplified DNA fragments (578 bp) predicted from the rat epidymis fat tissue - derived ob gene cDNA sequence. This DNA fragment was recovered from the gel by a known method, inserted into the plasmid vector pBluescript^{R} II SK+ (STRATAGENE, CA, USA), and introduced into Escherichia coli JM109 to obtain a transformant. Using this transformant, the base sequence of the DNA fragment (SEQ ID NO: 4) was determined. As a result, it was confirmed that the base sequence was identical with that of the cDNA fragment containing the region encoding the above 184 bp rat ob gene cDNA. The plasmid containing this cDNA fragment was designated as pBSK-rob, and the transformant obtained by introducing this plasmid was designated as Escherichia coli JM109/pBSK-rob. From the base sequence of the resulting rat ob gene cDNA (SEQ ID NO: 4), its gene product polypeptide (SEQ ID NO: 2) composed of 167 amino acid residues was predicted.

### Example 2

### Expression of the rat ob gene cDNA in Escherichia coli MM294 (DE3)

To amplify the ob gene using the plasmid pBSK-rob as a template and a primer to which BamHI and NdeI sites are attached to its 5'-terminus, the following three primers were synthesized.
Sense Primer No. 9:
   5'-pTCATATGGTGCCTATCCACAAAGTC-3'
   (SEQ ID NO: 13)
Sense Primer No. 10
   5'-pCGGATCCACAAAGTCCAGGATGAC A-3'
   (SEQ ID NO: 14)
Antisense Primer No. 11
   5'-pTTGGATCCCTCAACATGATCCTCGG-3'
   (SEQ ID NO: 15)

The coding region of the mature protein was amplified using the plasmid pBSK-rob as a template and the above primer Nos. 9 and 11. The coding region of the mature protein having a deletion of 2 amino acid residues at the N-terminus was amplified using the same template and the above primer Nos. 10 and 11. Each amplified DNA fragment was inserted into the Escherichia coli expression plasmid vectors pET-3c and pET-3xc (Methods in Enzymology, ed. by D. V. Goeddel, vol. 185, p. 68 (1990), Academic Press) to obtain plasmids designated as pET-romb and pET-robfx, respectively. Escherichia coli MM294 (DE3) was transformed with pET-robm and pET-robfx to express the ob gene under the control of T7 promoter (Methods in Enzymology, vol. 185, p. 60 (1990)). The transformed Escherichia coli was cultivated by a known method. The resulting cells were sonicated, and centrifuged. The precipitate was subjected to SDS polyacrylamide gel electrophoresis (SDS-PAGE). The expression of the ob gene mature protein (hereinafter sometimes referred to as rOBm) was detected at about 15 kDal, and the expression of the ob gene fusion protein (hereinafter sometimes referred to as rOBfx) was detected at about 43 kDal. Both expressions were detected as specific bands stained with CBB. Because each expression product (rOBm and rOBfx) formed an inclusion body, it was recovered from the precipitation fraction of the sonicated transformant. The mature protein precipitation was suspended with 4M urea, and shaken at 4°C overnight for solubilization, and then dialyzed against 20 mM Tris-HCl buffer (pH 7.5) to remove urea. This liquid was subjected to HiLoad™ 16/60 Superdex 200 column (Pharmacia Biotech AB, Uppsala, Sweden) equilibrated with 20 mM Tris-HCl buffer (pH 7.5) to recover the expression product. The rOBm subjected to gel filtration was eluted to the site of about 35 kDal. When this sample was subjected to SDS-PAGE, CBB staining of the gel showed an about 15 kDal band under reducing conditions and an about 30 kDal band under non-reducing conditions.

### Example 3

### Preparation of antiserum against rOBfx

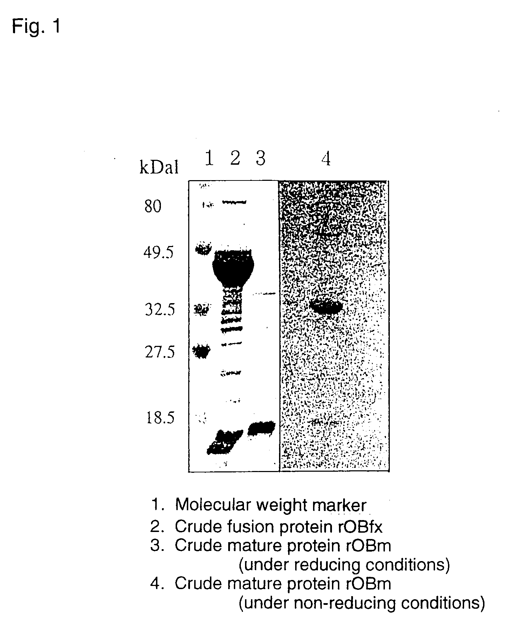

The crude rOBfx described in Example 2 was mixed with an equal volume of complete Freund's adjuvant. About 1 ml (containing 250 µg of the antigen) of the mixture was inoculated subcutaneously to the back of rabbits. Thereafter, a mixture of the crude rOBfx with an equal volume of incomplete Freund's adjuvant was inoculated subcutaneously to the back three times every 2 weeks. On the 7th day after the final inoculation, blood was drawn. The blood thus obtained was allowed to stand at 37°C for 30 minutes and then at 4°C overnight, and centrifuged to prepare rOBfx antiserum. The antiserum thus obtained was analyzed by the western blotting. As shown in Fig. 1, this antiserum recognizes both the fusion protein rOBfx (about 43 kDal) described in Example 2 (see lane 2) and the mature protein rOBm (about 30 kDal and about 15 kDal)(see lanes 3 and 4).

### Example 4

### Expression of rat ob gene cDNA in COS-7 cells

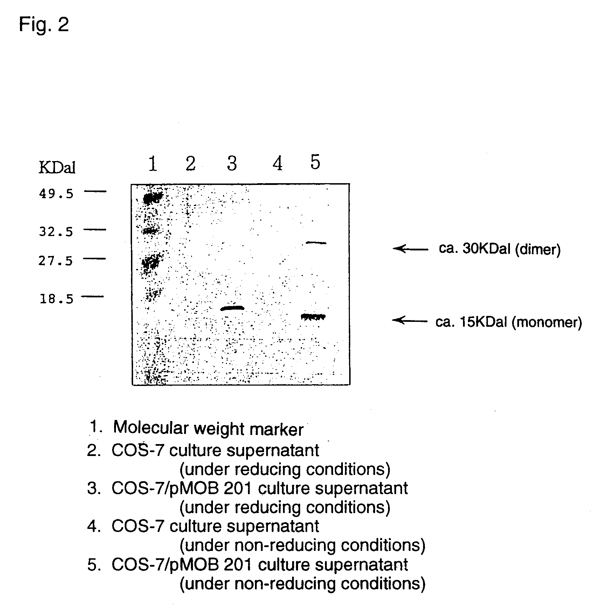

The rat ob gene cDNA cloning plasmid pBSK-rob was digested with EcoRI (Takara Shuzo Co., Ltd.) to obtain a 0.58kbp DNA fragment. This DNA fragment was treated with Klenow fragment (Takara Shuzo Co., Ltd.) to form blunt ends, and subcloned into SmaI site of the cloning vector pUC19 (Takara Shuzo Co., Ltd.) to obtain the plasmid p19-rob. This plasmid was digested with EcoRI and XbaI (Takara Shuzo Co., Ltd.) to obtain a 0.61 kbp DNA fragment. This DNA fragment was inserted into EcoRI-XbaI site of the animal cell expression vector pME18S (New Biochemistry Experimental Series (*Shin Seikagaku Jikken Kohza*), vol. 2, Nucleic Acid III, p. 95, ed. by the Japan Biochemical Society (1992)) to construct the rat ob gene cDNA animal cell expression plasmid pROB 201. This plasmid was purified by the CsCl density gradient equilibrium centrifugation (Molecular Cloning, vol. 1, p. 1.42 (1989), Cold Spring Harbor Laboratory Press) and transfected to COS-7 cells by the DEAE dextran method (Experimental Medicine (*Jikken Igaku*), vol. 5, p. 1019 (1987)). After 3 days, the culture supernatant was obtained. The supernatant was concentrated with Molecut (NIHON MILLIPORE LTD.), the concentrate was subjected to SDS-PAGE, and then analyzed with the rOBfx antiserum by the western blotting. As shown in Fig. 2, the predicted product of about 15 kDal was detected under reducing conditions (see lane 3), and a presumably dimer product of about 30 kDal as well as the predicted product of about 15 kDal were detected under non-reducing conditions (see lane 5).

As described above, the rat ob gene and its gene product obtained in the invention can be used to elucidate obesity mechanisms and diagnose obesity.

For example, the rat ob gene obtained by the invention can be used as a DNA probe to determine the content of the ob gene mRNA expressed in the living body by the known Northern blotting method, and thus can be used to diagnose obesity factors. The ob gene is introduced into prokaryotic or eukaryotic cells to obtain a transformant, and the gene product can be expressed in the transformant by gene engineering techniques to obtain a large amount of the ob gene product in high purity.

In addition, the anti - ob gene product antibody prepared using the rat ob gene product obtained by the invention can be used to detect and determine the ob gene product in the living body by the fluorescent antibody technique and the Western blotting method, and thus can be used to diagnose obesity factors associated with ob gene abnormalities.

(1) INFORMATION FOR SEQ ID NO: 1
(i) SEQUENCE CHARACTERISTICS
(A) LENGTH: 139
(B) TYPE: amino acid
(C) STRANDEDNESS: single
(D) TOPOLOGY: linear
(ii) MOLECULAR TYPE: peptide
(iii) SEQUENCE DESCRIPTION: SEQ ID NO: 1
His Lys Val Gln Asp Asp Thr Lys Thr Leu Ile Lys Thr Ile Val
1               5                   10                  15
Thr Arg Ile Asn Asp Ile Ser His Thr Gln Ser Val Ser Ala Arg
                20                  25                  30
Gln Arg Val Thr Gly Leu Asp Phe Ile Pro Gly Leu His Pro Ile
                35                  40                  45
Leu Ser Leu Ser Lys Met Asp Gln Thr Leu Ala Val Tyr Gln Gln
                50                  55                  60
Ile Leu Thr Ser Leu Pro Ser Gln Asn Val Leu Gln Ile Ala His
                65                  70                  75
Asp Leu Glu Asn Leu Arg Asp Leu Leu His Leu Leu Ala Phe Ser
                80                  85                  90
Lys Ser Cys Ser Leu Pro Gln Thr Arg Gly Leu Gln Lys Pro Glu
                95                  100                 105
Ser Leu Asp Gly Val Leu Glu Ala Ser Leu Tyr Ser Thr Glu Val
                110                 115                 120

Val Ala Leu Ser Arg Leu Gln Gly Ser Leu Gln Asp Ile Leu Gln
                125                 130                 135
Gln Leu Asp Leu.

(2) INFORMATION FOR SEQ ID NO: 2
(i) SEQUENCE CHARACTERISTICS
(A) LENGTH: 167
(B) TYPE: amino acid
(C) STRANDEDNESS: single
(D) TOPOLOGY: linear
(ii) MOLECULAR TYPE: peptide
(iii) SEQUENCE DESCRIPTION: SEQ ID NO: 2
X Cys Trp Arg Pro Leu Cys Arg Phe Leu Trp Leu Trp Ser Tyr
1 5 10 15
Leu Ser Tyr Val Gln Ala Val Pro Ile His Lys Val Gln Asp Asp
20 25 30
Thr Lys Thr Leu Ile Lys Thr Ile Val Thr Arg Ile Asn Asp Ile
35 40 45
Ser His Thr Gln Ser Val Ser Ala Arg Gln Arg Val Thr Gly Leu
50 55 60
Asp Phe Ile Pro Gly Leu His Pro Ile Leu Ser Leu Ser Lys Met
65 70 75
Asp Gln Thr Leu Ala Val Tyr Gln Gln Ile Leu Thr Ser Leu Pro
80 85 90

Ser Gln Asn Val Leu Gln Ile Ala His Asp Leu Glu Asn Leu Arg
95 100 105
Asp Leu Leu His Leu Leu Ala Phe Ser Lys Ser Cys Ser Leu Pro
110 115 120
Gln Thr Arg Gly Leu Gln Lys Pro Glu Ser Leu Asp Gly Val Leu
125 130 135
Glu Ala Ser Leu Tyr Ser Thr Glu Val Val Ala Leu Ser Arg Leu
140 145 150
Gln Gly Ser Leu Gln Asp Ile Leu Gln Gln Leu Asp Leu Ser Pro
155 160 165
Glu Cys.

wherein X is Met or hydrogen.
(3) INFORMATION FOR SEQ ID NO: 3
(i) SEQUENCE CHARACTERISTICS
(A) LENGTH: 417
(B) TYPE: nucleic acid
(C) STRANDEDNESS: double
(D) TOPOLOGY: linear
(ii) MOLECULAR TYPE: cDNA to mRNA
(iii) ORIGINAL SOURCE:
(A) ORGANISM: rat
(B) ORGANELLE: fat tissue

(iv) SEQUENCE DESCRIPTION: SEQ ID NO: 3
CACAAAGTCC AGGATGACAC CAAAACCCTC ATCAAGACCA TTGTCACCAG 50
GATCAATGAC ATTTCACACA CGCAGTCGGT ATCCGCCAGG CAGAGGGTCA 100
CCGGTTTGGA CTTCATTCCC GGGCTTCACC CCATTCTGAG TTTGTCCAAG 150
ATGGACCAGA CCCTGGCAGT CTATCAACAG ATCCTCACCA GCTTGCCTTC 200
CCAAAACGTG CTGCAGATAG CTCATGACCT GGAGAACCTG CGAGACCTCC 250
TCCATCTGCT GGCCTTCTCC AAGAGCTGCT CCCTGCCGCA GACCCGTGGC 300
CTGCAGAAGC CAGAGAGCCT GGATGGCGTC CTGGAAGCCT CGCTCTACTC 350
CACAGAGGTG GTGGCTCTGA GCAGGCTGCA GGGCTCTCTG CAGGACATTC 400
TTCAACAGTT GGACCTT 417
(4) INFORMATION FOR SEQ ID NO: 4
(i) SEQUENCE CHARACTERISTICS
(A) LENGTH: 501
(B) TYPE: nucleic acid
(C) STRANDEDNESS: double
(D) TOPOLOGY: linear
(ii) MOLECULAR TYPE: cDNA to mRNA
(iii) ORIGINAL SOURCE:
(A) ORGANISM: rat
(B) ORGANELLE: fat tissue
(iv) SEQUENCE DESCRIPTION: SEQ ID NO: 4
ATGTGCTGGA GACCCCTGTG CCGGTTCCTG TGGCTTTGGT CCTATCTGTC 50
CTATGTTCAA GCTGTGCCTA TCCACAAAGT CCAGGATGAC ACCAAAACCC 100
TCATCAAGAC CATTGTCACC AGGATCAATG ACATTTCACA CACGCAGTCG 150
GTATCCGCCA GGCAGAGGGT CACCGGTTTG GACTTCATTC CCGGGCTTCA 200

CCCCATTCTG AGTTTGTCCA AGATGGACCA GACCCTGGCA GTCTATCAAC 250
AGATCCTCAC CAGCTTGCCT TCCCAAAACG TGCTGCAGAT AGCTCATGAC 300
CTGGAGAACC TGCGAGACCT CCTCCATCTG CTGGCCTTCT CCAAGAGCTG 350
CTCCCTGCCG CAGACCCGTG GCCTGCAGAA GCCAGAGAGC CTGGATGGCG 400
TCCTGGAAGC CTCGCTCTAC TCCACAGAGG TGGTGGCTCT GAGCAGGCTG 450
CAGGGCTCTC TGCAGGACAT TCTTCAACAG TTGGACCTTA GCCCTGAATG 500
C 501
(5) INFORMATION FOR SEQ ID NO: 5
(i) SEQUENCE CHARACTERISTICS
(A) LENGTH: 20
(B) TYPE: nucleic acid
(C) STRANDEDNESS: single
(ii) MOLECULAR TYPE: sense
(iii) SEQUENCE DESCRIPTION: SEQ ID NO: 5
AATGTGCTGG AGACCCCTGT 20
(6) INFORMATION FOR SEQ ID NO: 6
(i) SEQUENCE CHARACTERISTICS
(A) LENGTH: 20
(B) TYPE: nucleic acid
(C) STRANDEDNESS: single
(ii) MOLECULAR TYPE: antisense
(iii) SEQUENCEED DESCRIPTION: SEQ ID NO: 6
CAGCATTCAG GGCTAACATC 20

(7) INFORMATION FOR SEQ ID NO: 7
(i) SEQUENCE CHARACTERISTICS
(A) LENGTH: 18
(B) TYPE: nucleic acid
(C) STRANDEDNESS: single
(ii) MOLECULAR TYPE: antisense
(iii) SEQUENCE DESCRIPTION: SEQ ID NO: 7
ATCCTGGTGA CAATGGTC 18
(8) INFORMATION FOR SEQ ID NO: 8
(i) SEQUENCE CHARACTERISTICS
(A) LENGTH: 18
(B) TYPE: nucleic acid
(C) STRANDEDNESS: single
(ii) MOLECULAR TYPE: antisense
(iii) SEQUENCEED DESCRIPTION: SEQ ID NO: 8
CTGTTGATAG ACTGCCAG 18
(9) INFORMATION FOR SEQ ID NO: 9
(i) SEQUENCE CHARACTERISTICS
(A) LENGTH: 20
(B) TYPE: nucleic acid
(C) STRANDEDNESS: single
(ii) MOLECULAR TYPE: sense
(iii) SEQUENCE DESCRIPTION: SEQ ID NO: 9
TGCTCCAGCA GCTGCAAGGT 20

(10) INFORMATION FOR SEQ ID NO: 10
(i) SEQUENCE CHARACTERISTICS
(A) LENGTH: 25
(B) TYPE: nucleic acid
(C) STRANDEDNESS: single
(ii) MOLECULAR TYPE: sense
(iii) SEQUENCE DESCRIPTION: SEQ ID NO: 10
CAAGAGGAAT TCCCCAGCGA GGAAA 25
(11) INFORMATION FOR SEQ ID NO: 11
(i) SEQUENCE CHARACTERISTICS
(A) LENGTH: 20
(B) TYPE: nucleic acid
(C) STRANDEDNESS: single
(ii) MOLECULAR TYPE: antisense
(iii) SEQUENCEED DESCRIPTION: SEQ ID NO: 11
TGAGGATACC TGGGAGCCAA 20
(12) INFORMATION FOR SEQ ID NO: 12
(i) SEQUENCE CHARACTERISTICS
(A) LENGTH: 25
(B) TYPE: nucleic acid
(C) STRANDEDNESS: single
(ii) MOLECULAR TYPE: antisense
(iii) SEQUENCE DESCRIPTION: SEQ ID NO: 12
GGGAATTCCC TCAACATGAT CCTCG 25

(13) INFORMATION FOR SEQ ID NO: 13
(i) SEQUENCE CHARACTERISTICS
(A) LENGTH: 25
(B) TYPE: nucleic acid
(C) STRANDEDNESS: single
(ii) MOLECULAR TYPE: sense
(iii) SEQUENCEED DESCRIPTION: SEQ ID NO: 13
TCATATGGTG CCTATCCACA AAGTC 25
(14) INFORMATION FOR SEQ ID NO: 14
(i) SEQUENCE CHARACTERISTICS
(A) LENGTH: 25
(B) TYPE: nucleic acid
(C) STRANDEDNESS: single
(ii) MOLECULAR TYPE: sense
(iii) SEQUENCE DESCRIPTION: SEQ ID NO: 14
CGGATCCACA AAGTCCAGGA TGACA 25
(15) INFORMATION FOR SEQ ID NO: 15
(i) SEQUENCE CHARACTERISTICS
(A) LENGTH: 25
(B) TYPE: nucleic acid
(C) STRANDEDNESS: single
(ii) MOLECULAR TYPE: antisense
(iii) SEQUENCE DESCRIPTION: SEQ ID NO: 15
TTGGATCCCT CAACATGATC CTCGG 25

SEQUENCE LISTING

(1) GENERAL INFORMATION:

(i) APPLICANT:
(A) NAME: Takeda Chemical Industries, Ltd.
(B) STREET: 1-1, Doshomachi 4-chome, Chuo-ku
(C) CITY: Osaka 541
(E) COUNTRY: Japan
(F) POSTAL CODE (ZIP): none

(ii) TITLE OF INVENTION: Rat Obese Gene, its Gene Product and its
Production

(iii) NUMBER OF SEQUENCES: 15

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 139 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 1:

His Lys Val Gln Asp Asp Thr Lys Thr Leu Ile Lys Thr Ile Val Thr
1               5                   10                  15

Arg Ile Asn Asp Ile Ser His Thr Gln Ser Val Ser Ala Arg Gln Arg
            20                  25                  30

Val Thr Gly Leu Asp Phe Ile Pro Gly Leu His Pro Ile Leu Ser Leu
        35                  40                  45

Ser Lys Met Asp Gln Thr Leu Ala Val Tyr Gln Gln Ile Leu Thr Ser
    50                  55                  60

Leu Pro Ser Gln Asn Val Leu Gln Ile Ala His Asp Leu Glu Asn Leu
65                  70                  75                  80

Arg Asp Leu Leu His Leu Leu Ala Phe Ser Lys Ser Cys Ser Leu Pro

                85                  90                  95

Gln Thr Arg Gly Leu Gln Lys Pro Glu Ser Leu Asp Gly Val Leu Glu
            100                 105                 110

Ala Ser Leu Tyr Ser Thr Glu Val Val Ala Leu Ser Arg Leu Gln Gly
        115                 120                 125

Ser Leu Gln Asp Ile Leu Gln Gln Leu Asp Leu
    130                 135

(2) INFORMATION FOR SEQ ID NO: 2:

(i) SEQUENCE CHARACTERISTICS:
(A) LENGTH: 167 amino acids
(B) TYPE: amino acid
(C) STRANDEDNESS: single
(D) TOPOLOGY: linear

(ii) MOLECULE TYPE: peptide

(ix) FEATURE:
(A) NAME/KEY: Region
(B) LOCATION:1
(D) OTHER INFORMATION:/note= "Xaa is Met or hydrogen"

(xi) SEQUENCE DESCRIPTION: SEQ ID NO: 2:

Xaa Cys Trp Arg Pro Leu Cys Arg Phe Leu Trp Leu Trp Ser Tyr Leu
1               5                   10                  15

Ser Tyr Val Gln Ala Val Pro Ile His Lys Val Gln Asp Asp Thr Lys
            20                  25                  30

Thr Leu Ile Lys Thr Ile Val Thr Arg Ile Asn Asp Ile Ser His Thr
        35                  40                  45

Gln Ser Val Ser Ala Arg Gln Arg Val Thr Gly Leu Asp Phe Ile Pro
    50                  55                  60

Gly Leu His Pro Ile Leu Ser Leu Ser Lys Met Asp Gln Thr Leu Ala
65                  70                  75                  80

Val Tyr Gln Gln Ile Leu Thr Ser Leu Pro Ser Gln Asn Val Leu Gln
                85                  90                  95

Ile Ala His Asp Leu Glu Asn Leu Arg Asp Leu Leu His Leu Leu Ala
            100                 105                 110

Phe Ser Lys Ser Cys Ser Leu Pro Gln Thr Arg Gly Leu Gln Lys Pro
        115                 120                 125

Glu Ser Leu Asp Gly Val Leu Glu Ala Ser Leu Tyr Ser Thr Glu Val

130                    135                    140

Val Ala Leu Ser Arg Leu Gln Gly Ser Leu Gln Asp Ile Leu Gln Gln
145                 150                 155                 160

Leu Asp Leu Ser Pro Glu Cys
                165

(2) INFORMATION FOR SEQ ID NO: 3:

(i) SEQUENCE CHARACTERISTICS:
(A) LENGTH: 417 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: rat
(F) TISSUE TYPE: fat tissue

(xi) SEQUENCE DESCRIPTION: SEQ ID NO: 3:

CACAAAGTCC AGGATGACAC CAAAACCCTC ATCAAGACCA TTGTCACCAG GATCAATGAC 60

ATTTCACACA CGCAGTCGGT ATCCGCCAGG CAGAGGGTCA CCGGTTTGGA CTTCATTCCC 120

GGGCTTCACC CCATTCTGAG TTTGTCCAAG ATGGACCAGA CCCTGGCAGT CTATCAACAG 180

ATCCTCACCA GCTTGCCTTC CCAAAACGTG CTGCAGATAG CTCATGACCT GGAGAACCTG 240

CGAGACCTCC TCCATCTGCT GGCCTTCTCC AAGAGCTGCT CCCTGCCGCA GACCCGTGGC 300

CTGCAGAAGC CAGAGAGCCT GGATGGCGTC CTGGAAGCCT CGCTCTACTC CACAGAGGTG 360

GTGGCTCTGA GCAGGCTGCA GGGCTCTCTG CAGGACATTC TTCAACAGTT GGACCTT 417

(2) INFORMATION FOR SEQ ID NO: 4:

(i) SEQUENCE CHARACTERISTICS:
(A) LENGTH: 501 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: rat
(F) TISSUE TYPE: fat tissue

(xi) SEQUENCE DESCRIPTION: SEQ ID NO: 4:

ATGTGCTGGA GACCCCTGTG CCGGTTCCTG TGGCTTTGGT CCTATCTGTC CTATGTTCAA 60

GCTGTGCCTA TCCACAAAGT CCAGGATGAC ACCAAAACCC TCATCAAGAC CATTGTCACC 120

AGGATCAATG ACATTTCACA CACGCAGTCG GTATCCGCCA GGCAGAGGGT CACCGGTTTG 180

GACTTCATTC CCGGGCTTCA CCCCATTCTG AGTTTGTCCA AGATGGACCA GACCCTGGCA 240

GTCTATCAAC AGATCCTCAC CAGCTTGCCT TCCCAAAACG TGCTGCAGAT AGCTCATGAC 300

CTGGAGAACC TGCGAGACCT CCTCCATCTG CTGGCCTTCT CCAAGAGCTG CTCCCTGCCG 360

CAGACCCGTG GCCTGCAGAA GCCAGAGAGC CTGGATGGCG TCCTGGAAGC CTCGCTCTAC 420

TCCACAGAGG TGGTGGCTCT GAGCAGGCTG CAGGGCTCTC TGCAGGACAT TCTTCAACAG 480

TTGGACCTTA GCCCTGAATG C 501

(2) INFORMATION FOR SEQ ID NO: 5:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(iv) ANTI-SENSE: NO

(xi) SEQUENCE DESCRIPTION: SEQ ID NO: 5:

AATGTGCTGG AGACCCCTGT 20

(2) INFORMATION FOR SEQ ID NO: 6:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(iv) ANTI-SENSE: YES

(xi) SEQUENCE DESCRIPTION: SEQ ID NO: 6:

CAGCATTCAG GGCTAACATC 20

(2) INFORMATION FOR SEQ ID NO: 7:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid

(iv) ANTI-SENSE: YES

(xi) SEQUENCE DESCRIPTION: SEQ ID NO: 7:

ATCCTGGTGA CAATGGTC 18

(2) INFORMATION FOR SEQ ID NO: 8:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid

(iv) ANTI-SENSE: YES

(xi) SEQUENCE DESCRIPTION: SEQ ID NO: 8:

CTGTTGATAG ACTGCCAG 18

(2) INFORMATION FOR SEQ ID NO: 9:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(iv) ANTI-SENSE: NO

(xi) SEQUENCE DESCRIPTION: SEQ ID NO: 9:

TGCTCCAGCA GCTGCAAGGT 20

(2) INFORMATION FOR SEQ ID NO: 10:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid

(iv) ANTI-SENSE: NO

(xi) SEQUENCE DESCRIPTION: SEQ ID NO: 10:

CAAGAGGAAT TCCCCAGCGA GGAAA 25

(2) INFORMATION FOR SEQ ID NO: 11:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(iv) ANTI-SENSE: YES

(xi) SEQUENCE DESCRIPTION: SEQ ID NO: 11:

TGAGGATACC TGGGAGCCAA 20

(2) INFORMATION FOR SEQ ID NO: 12:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid

(iv) ANTI-SENSE: YES

(xi) SEQUENCE DESCRIPTION: SEQ ID NO: 12:

GGGAATTCCC TCAACATGAT CCTCG 25

(2) INFORMATION FOR SEQ ID NO: 13:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid

(iv) ANTI-SENSE: NO

(xi) SEQUENCE DESCRIPTION: SEQ ID NO: 13:

TCATATGGTG CCTATCCACA AAGTC 25

(2) INFORMATION FOR SEQ ID NO: 14:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid

(iv) ANTI-SENSE: NO

(xi) SEQUENCE DESCRIPTION: SEQ ID NO: 14:

CGGATCCACA AAGTCCAGGA TGACA 25

(2) INFORMATION FOR SEQ ID NO: 15:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid

(iv) ANTI-SENSE: YES

(xi) SEQUENCE DESCRIPTION: SEQ ID NO: 15:

TTGGATCCCT CAACATGATC CTCGG 25

## Claims

1. A polypeptide which has an amino acid sequence comprising the amino acid sequence shown in SEQ ID NO: 1.

2. A polypeptide which has an amino acid sequence comprising the amino acid sequence shown in SEQ ID NO: 2.

3. A polypeptide according to Claim 1 or 2, which is a gene product coding for rat obese cDNA.

4. A recombinant DNA encoding a polypeptide according to Claim 1 or 2.

5. A recombinant DNA according to Claim 4, which comprises the DNA sequence shown in SEQ ID NO: 3.

6. A recombinant DNA according to Claim 4, which comprises the DNA sequence shown in SEQ ID NO: 4.

7. A vector comprising a recombinant DNA according to Claim 4.

8. A host cell transformed with a recombinant DNA according to Claim 4.

9. A host cell according to Claim 8, wherein the cell is an Escherichia coli cell.

10. A method for producing a polypeptide according to Claim 1 or 2, which comprises the steps of culturing a host cell according to Claim 8 in a culture medium, accumulating the polypeptide in the culture and recovering the same.

11. An antibody which reacts to a gene product coding for rat obese cDNA.
